# EUROPÄISCHE PATENTSCHRIFT

(11) **EP 0 870 475 B1**
(45) Veröffentlichungstag und Bekanntmachung des Hinweises auf die Patenterteilung: **03.12.2003**
(21) Anmeldenummer: 98104689.9
(22) Anmeldetag: 16.03.1998
(51) Int. Cl.: A61B 19/00, F16C 11/10, A47C 1/03

(54) **Arztstuhl**
Doctor's chair
Chaise pour médecins

(30) Priorität: 11.04.1997 DE 19715303
(43) Veröffentlichungstag der Anmeldung: 14.10.1998
(73) Patentinhaber: Erich Edlinger KG, 72213 Altensteig (DE)
(72) Erfinder: Edlinger, Erich, 72227 Egenhausen (DE)
(74) Vertreter: Gleiss, Alf-Olav, Dr.jur. Dipl.-Ing.

(56) Entgegenhaltungen:
- EP-A- 0 696 442
- DE-U- 29 511 900
- FR-A- 2 339 936
- US-A- 3 124 328
- US-A- 3 278 207
- US-A- 4 655 501
- US-A- 5 281 001

## Beschreibung

Die Erfindung betrifft einen Arztstuhl mit mindestens einer am Arztstuhl angebrachten in mindestens zwei Achsen verstellbaren Armauflage sowie mit einer Verstell- und Fixiereinrichtung.

Arztstühle der hier genannten Art sind bekannt (EP-A-0 868 885; US-A 3 124 328). Sie werden allgemein von Ärzten benutzt, die Präzisionsarbeit leisten müssen, beispielsweise Zahn- und Augenärzten, die ihren Arm während der Behandlung eines Patienten aufstützen. Die dabei verwendete Armauflage muß dabei in eine optimale Arbeitsposition verlagerbar und in dieser fixierbar sein. Verstellbare Armauflagen sind bekannt. Sie weisen mit der Hand bedienbare Feststelleinrichtungen auf, die die Armauflage in der gewünschten Position fixieren sollen. Je mehr Bewegungsmöglichkeiten gegeben sind, um so mehr Feststelleinrichtungen sind vorhanden. Es ist also sehr aufwendig, die Armauflage in die gewünschte Position zu bringen. Darüber hinaus hat sich gezeigt, daß das von dem Arzt eingestellte Feststellmoment nicht immer gleich ist. Beispielsweise mit feuchten Händen kann dieser nur ein geringeres Feststellmoment aufbringen, als dies mit trockenen Händen der Fall ist. Es ist daher möglich, daß eine Armauflage während der Behandlung eines Patienten aus der vorgegebenen Position verschwenkt wird, was zu schweren Verletzungen des Patienten führen kann.

Ein Arzt, insbesondere ein Zahnarzt, befindet sich während der Patientenbehandlung in einer Haltung, in der er seinen Oberkörper aus eigener Kraft in einer nach vornübergebeugten, verdrehten Position zu halten hat. Zusätzlich zu dieser angespannten Zwangshaltung, die der Arzt einnimmt, um sich in eine optimale Behandlungsposition zu bringen, verstärkt sich die Belastung des Arztes dadurch, daß dieser den üblicherweise verfahrbaren Arztstuhl mit seinen Füßen am Fußboden fixieren muß. Diese Gesamtbelastung führt auf Dauer unter Umständen zu erheblichen gesundheitlichen Problemen (Langzeitschäden). Diese Schäden erstrecken sich insbesondere auf die Rücken- und Halsmuskulatur, auf die Wirbelsäule, insbesondere im Bereich der Lendenund Halswirbelsäule, den Nierenbereich, sowie auf den Schulter-, Nacken- und Kopfbereich.

Es ist daher Aufgabe der Erfindung, einen Arztstuhl zu schaffen, der diese Nachteile nicht aufweist.

Zur Lösung dieser Aufgabe wird ein Arztstuhl vorgeschlagen, der die in Anspruch 1 genannten Merkmale aufweist. Der Stuhl zeichnet sich dadurch aus, daß er bezüglich jeder Achse, in der die Armauflage verstellbar ist, eine Blockiereinrichtung aufweist, die einen federbelasteten, eine definierte Blokkierkraft aufbauenden Blockierbacken aufweist. Die Blockiereinrichtungen sind so miteinander gekoppelt, daß die Bewegung der Armauflage bezüglich der zwei Achsen blockiert und freigegeben wird. In der Freigabeposition kann der Arzt die Armlehne optimal ausrichten. In der Blockierposition der Blockiereinrichtungen, wird die Armauflage mit Hilfe der definierten Blockierkraft in der gewünschten Position gehalten. Es ist also praktisch ausgeschlossen, daß sich die Armauflage während der Behandlung eines Patienten verlagert.

Bevorzugt wird ein Ausführungsbeispiel des Arztstuhls, das sich dadurch auszeichnet, daß einer Bewegung bezüglich einer dritten Achse der Armauflage eine separate Blockiereinrichtung zugeordnet ist, die einen dritten Blockierbacken aufweist. Auch dieser ist federbelastet und baut eine definierte Blockierkraft auf. Er ist mit der ersten und zweiten Blockiereinrichtung so gekoppelt, daß die Bewegung der Armauflage bezüglich der dritten Achse gemeinsam mit den Bewegungen bezüglich der anderen Achsen blockiert und freigegeben wird. Es ist also möglich, eine Längsverlagerung der Armauflage, eine Drehbewegung und eine Höhenverstellung gleichzeitig vorzunehmen und eine gemeinsame Festlegung aller Blockiereinrichtungen zu erreichen, so daß die Armauflage sicher in einer gewünschten Position festgehalten wird. Eine Gefährdung des Patienten ist auch bei dieser Ausgestaltung des Arztstuhls praktisch ausgeschlossen.

Bevorzugt wird schließlich eine Ausführungsform des Arztstuhls, bei der mindestens einer vorzugsweise allen Blockierbacken ein Zahnelement zugeordnet ist, das im blockierten Zustand einen Formschluß ermöglicht. Es ist damit auch möglich, bei einem Bruch der die Blockierbacken vorbelastenden Federelemente eine definierte Position der Armauflage beizubehalten, das heißt einen definierten Eingriff zwischen Blockierbacken und Zahnelement aufrechtzuerhalten und damit auch unter diesen Betriebsbedingungen eine definierte Position der Armauflage sicherzustellen. Damit ist die Sicherheit für einen Patienten noch weiter erhöht.

Weitere Ausgestaltungen ergeben sich aus den übrigen Unteransprüchen.

Die Erfindung wird im folgenden anhand der Zeichnung näher erläutert. Es zeigen:
- Figur 1: eine Prinzipskizze eines Arztstuhls mit einer Armauflage;
- Figur 2: eine Prinzipskizze einer Armauflage und
- Figur 3: eine Prinzipskizze einer zusätzlichen Blockiereinrichtung für eine in Figur 2 dargestellte Armauflage.

Der im folgenden beschriebene Arztstuhl ist grundsätzlich für alle Ärzte verwendbar, die feinste Präzisionsarbeiten durchführen müssen und dabei ihren Arm auf einer Armauflage abstützen. Er ist beispielsweise für Augenärzte aber auch für Zahnärzte sehr gut verwendbar. Im folgenden wird rein beispielhaft davon ausgegangen, daß es sich hier um einen Zahnarztstuhl handelt.

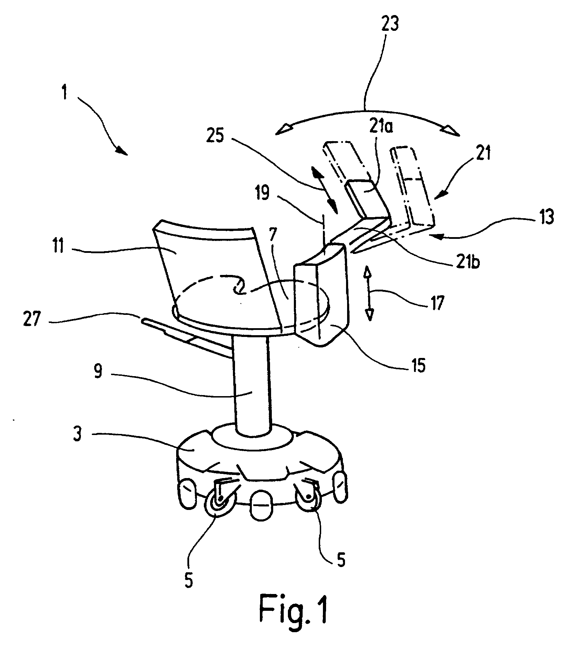

Bei dem in Figur 1 dargestellten Ausführungsbeispiel eines Arztstuhls handelt es sich um einen Stuhl mit einem Fahrgestell 3 mit einer Anzahl von Rädern 5. Angesprochen sind hier aber auch Arztstühle, die auf einem Schienensystem verlagerbar sind, beispielsweise auf einem drehbar an einem Untergrund, beispielsweise am Boden einer Praxis befestigten Schwenkarm.

Der Arztstuhl 1 weist eine Sitzfläche 7 auf, die sich über eine Sitzsäule 9 am Fahrgestell 3 abstützt. An der Sitzfläche 3 kann eine Rückenlehne 11 vorgesehen sein. Wesentlich ist hier, daß an dem Arztstuhl 1, hier beispielhaft an der Sitzfläche 7 eine Armauflage 13 vorgesehen ist. Diese ist über eine Tragsäule 15 an der Sitzfläche 7 höhenverstellbar befestigt, was durch einen Doppelpfeil 17 angedeutet ist. Die Armauflage umfaßt eine an der Tragsäule 15 drehbar um eine Drehachse 19 gelagerte Armstütze 21 auf, die zwei hier beispielhaft in einer Ebene angeordnete Stützelemente 21a und 21b umfaßt. Die um die Drehachse 19 mögliche Schwenkbewegung der Armstütze 21 ist durch einen Doppelpfeil 23 angedeutet. Das Stützelement 21a ist in Richtung seiner Längsausdehnung verlagerbar, was durch einen Doppelpfeil 25 angedeutet ist.

In Figur 1 sind zwei mögliche Positionen der Armstütze 21 angedeutet. In beiden Fällen ist gestrichelt dargestellt, daß das Stützelement 21a längsverlagerbar beziehungsweise ausziehbar ist.

Ein Arzt kann also die Armauflage 13 des Arztstuhls 1 in eine für die Behandlung eines Patienten optimale Position bringen und dabei die Armauflage in mindestens zwei Achsen verstellen. Es ist hier also eine Längsverschiebbarkeit des Stützelements 21a, also eine Bewegung bezüglich einer ersten Achse, eine Schwenkbarkeit der Armstütze 21 um die Drehachse 19, also eine Bewegung um eine zweite Achse möglich. Schließlich ist noch eine Höheneinstellbarkeit in Richtung der Drehachse möglich, damit eine Bewegung in Richtung einer dritten Achse, die hier mit der Drehachse 19 zusammenfällt.

Der Vollständigkeit halber sei hier noch daraufhin verwiesen, daß der gesamte Arztstuhl höhenverstellbar ausgelegt sein kann, daß also die Sitzfläche 7 gegenüber dem Fahrgestell in der Höhe variabel ist. Die Verstellung kann beispielsweise mit Hilfe eines Handhebels 27 erfolgen, der hier lediglich angedeutet und grundsätzlich bekannt ist.

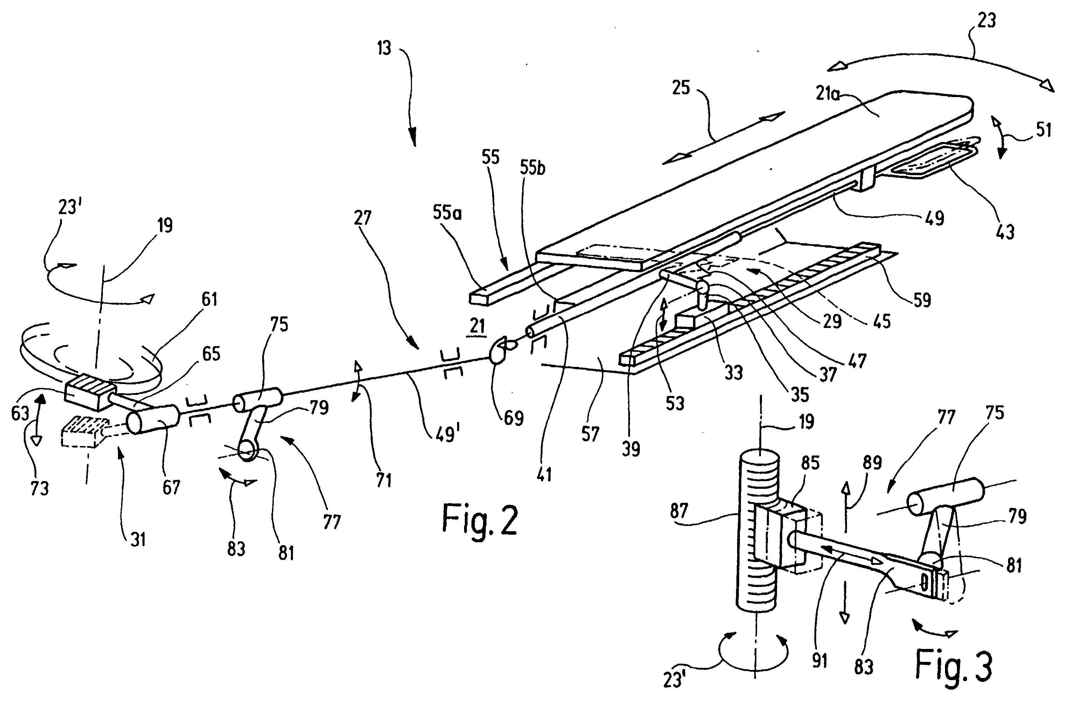

Figur 2 zeigt eine Prinzipskizze der Armauflage 13. Gleiche Teile sind mit gleichen Bezugsziffern versehen, so daß auf die Erläuterungen zu Figur 1 verwiesen wird. In Figur 2 wurden weite Teile der Armauflage 13 weggelassen, um eine Verstell- und Fixiereinrichtung 27 besser erkennbar zu machen. Diese wird im folgenden kurz als Einrichtung 27 bezeichnet.

Die Einrichtung 27 umfaßt eine erste Blockiereinrichtung 29, die die axiale Verlagerbarkeit des Stützelements 21a gemäß dem Doppelpfeil 25 blokkiert beziehungsweise freigibt. Eine zweite Blokkiereinrichtung 31 beeinflußt die Drehbarkeit der Armstütze 21 um die Drehachse 19, die durch einen weiteren Doppelpfeil 23' angedeutet ist. Durch diesen Doppelpfeil 23' wird die Drehung beziehungsweise Schwenkung der Armstütze 21 um die Drehachse 19 noch einmal verdeutlicht. Die erste Blockiereinrichtung 29 umfaßt einen ersten Blockierbacken 33, der über einen hier vertikal verlaufenden Blockierstift 35 und eine gelenkige Verbindung 37 sowie über einen Drehhebel 39 mit einem Verstellrohr 41 verbunden ist. Dieses kann über einen hier als Handhebel 43 ausgebildeten Betätigungshebel gegen die Kraft eines an einem Widerlager 45 abgestützten Federelements 47 verschwenkt werden. Das Verstellrohr 41 ist hier über eine Verstellwelle 49 mit dem Verstellrohr 41 gekoppelt. Die Verstellwelle ist vorzugsweise als Vielkeilwelle beziehungsweise - profil ausgebildet und formschlüssig innerhalb des Verstellrohrs 41 geführt. Das heißt, ein über den Handhebel 43 in die Verstellwelle 49 eingeleitetes Torsionsmoment wird auf das Verstellrohr 41 übertragen. Gleichzeitig ist sichergestellt, daß das Verstellrohr 41 in Richtung des Doppelpfeils 25 auf der Verstellwelle 49 hin und her gleiten kann, wobei in allen Funktionsstellungen die Übertragung eines Torsionsmoments von der Verstellwelle 49 auf das Verstellrohr 41 und umgekehrt möglich ist.

Wird der Handhebel 43 aus der mit durchgezogenen Linien dargestellten Blockierposition entgegen dem Uhrzeigersinn nach oben verschwenkt und damit in seine gestrichelt dargestellte Entriegelungsposition bewegt, so findet eine Drehung der Verstellwelle 49 im Gegenuhrzeigersinn statt, gleichzeitig wird das Verstellrohr 41 in gleicher Richtung bewegt. Der starr mit dem Verstellrohr 41 gekoppelte Drehhebel 39 wird dadurch ebenfalls gegen den Uhrzeigersinn verschwenkt, so daß der Blockierstift 35 senkrecht nach oben verlagert wird. Dadurch wird die erste Blockierbacke 33 angehoben.

Die Bewegung des Handhebels 43 zwischen der Blokkier- und Freigabeposition ist durch einen Doppelpfeil 51 angedeutet, ebenso die Auf- und Abbewegung des ersten Blockierbackens 33 durch einen Doppelpfeil 53. Bei den Doppelpfeilen ist eine Pfeilspitze schwarz ausgefüllt, nämlich die, die die Bewegungsrichtung in die Blockierstellung wiedergibt. Entsprechend ist durch eine weiße nicht ausgefüllte Pfeilspitze die Bewegungsrichtung angedeutet, die der Handhebel 43 beziehungsweise der erste Blokkierbacken 33 vollführt, wenn er in die Entriegelungsposition verlagert wird.

Das Verstellrohr 41 ist mit dem Stützelement 21a der Armstütze 21 gekoppelt. Es wird also gemeinsam mit diesem in Richtung der Verstellwelle 49 hin und her bewegt, was durch den Doppelpfeil 25 angedeutet ist. Das erste Stützelement 21a ist mit einer geeigneten Linearführung 55 gekoppelt, die hier zwei parallel zueinander verlaufende Schienen 55a und 55b umfaßt. Anstelle der Schienen können auch ein oder zwei Teleskoparme vorgesehen werden, die das Stützelement 21a bei einer Verlagerung entsprechend dem Doppelpfeil 25 tragen.

Der erste Blockierbacken 33 wird bei der durch den schwarzen Pfeil angedeuteten Blockierposition aufgrund der von dem Federelement 47 ausgeübten Federkraft auf einen Untergrund 57 angepreßt, auf dem hier ein als Zahnstange 59 ausgebildetes Zahnelement ruht, welches parallel zum Bewegungspfad des ersten Blockierbackens 33 verläuft und bei einer Längsbewegung des Stützelements 21a entlang dem Doppelpfeil 25 in beliebigen Positionen mit dem ersten Blockierbacken 33 in Eingriff bringbar ist.

Eine Bewegung der Armauf lage 13 beziehungsweise des Stützelements 21a der Armstütze 21 in Richtung einer ersten Achse, nämlich in Richtung des Doppelpfeils 25 kann durch die erste Blockiereinrichtung 29 in jeder beliebigen Position unterbrochen werden. Es ist jeweils eine Blockierung des Stützelements 21a möglich.

An der hier nicht dargestellten Tragsäule 15 kann ein hier als Zahnkranz 61 ausgebildetes Zahnelement drehfest zur Drehachse 19 angebracht werden, das Teil der zweiten Blockiereinrichtung 31 ist. Diese umfaßt außerdem einen zweiten Blockierbacken 63, der über einen Drehhebel 65 mit einer Fortsetzung 49' über eine Stellhülse 67 drehfest gekoppelt ist. Die Fortsetzung 49' ist drehstarr mit der Verstellwelle 49 verbunden, das heißt eine Drehung der Verstellwelle 49 wird auf deren Fortsetzung 49' übertragen. Die Verstellwelle und deren Fortsetzung verlaufen hier unter einem stumpfen Winkel. Die Drehbewegung der Verstellwelle 49 wird auf deren Fortsetzung 49' über eine geeignete Gelenkverbindung, hier über ein Kreuzgelenk 69 übertragen. Eine Hin- und Herbewegung des Handhebels 43 entsprechend dem Doppelpfeil 51 führt also zu einer hin und her gehenden Drehbewegung der Fortsetzung 49' entsprechend dem Doppelpfeil 71. Eine Bewegung des Handhebels 43 gegen die Kraft des Federelements 47, die über das Kreuzgelenk 69 auf die Fortsetzung 49' übertragen wird, in Richtung der weißen Pfeilspitze des Doppelpfeils 51 führt zu einer Drehbewegung des Fortsatzes 49' im Gegenuhrzeigersinn, also ebenfalls in Richtung der weißen Pfeilspitze des Doppelpfeils 71, das heißt in Richtung der Entriegelungsposition. Bei einer Bewegung des Handhebels 43 nach oben wird der zweite Blockierbacken 63 von dem Zahnkranz 61, wie durch den Doppelpfeil 73 angedeutet, nach unten verschwenkt. Der zweite Blockierbacken 63 gibt also den Zahnkranz 61 frei, so daß also die Armstütze 21 gegenüber der Drehachse 19 und gegenüber dem feststehenden Zahnkranz 61 in Richtung des Doppelpfeils 23 beziehungsweise 23' verschwenkt werden kann. Durch die starre Kopplung der Verstellwelle 49 mit deren Fortsatz 49' wird gleichzeitig der erste Blockierbacken 31 von der Zahnstange 59 abgehoben und in die Entriegelungsposition gebracht.

Aus dem oben Gesagten wird deutlich, daß die erste Blockiereinrichtung 29 und die zweite Blockiereinrichtung 31 über die Verstellwelle 49, das Kreuzgelenk 69 und über den Fortsatz 49' der Verstellwelle drehstarr miteinander gekoppelt sind. Das heißt eine Verschwenkung des Handhebels 43 in die Entriegelungsposition entriegelt die erste Blockiereinrichtung 29 und die zweite Blockiereinrichtung 31. Wird der Handhebel 43 losgelassen und durch die Kraft des Federelements 47 in seine untere Blokkierposition verlagert, so werden damit gleichzeitig der erste Blockierbacken 33 und der zweite Blockierbacken 63 in seine Blockierposition verlagert.

Es wird also deutlich, daß die Verstell- und Fixiereinrichtung 27 gleichzeitig eine Längsbewegung des Stützelements 21a und eine Drehbewegung um die Drehachse 19 freigibt und blockiert.

Aus Figur 2 ist erkennbar, daß mit dem Fortsatz 49' eine weitere Kopplungshülse 75 zusammenwirkt, die Teil einer dritten Blockiereinrichtung 77 ist. Diese umfaßt einen starr mit der Kopplungshülse gekoppelten Drehhebel 79, der an seinem der Kopplungshülse 75 gegenüberliegenden freien Ende eine Gelenkverbindung 81 aufweist. Bei einer Schwenkbewegung des Fortsatzes 49' im Sinne des Doppelpfeils 71 findet eine Schwenkbewegung der Kopplungshülse 75 und des Drehhebels 79 gemäß dem Doppelpfeil 83 statt, wobei durch einen schwarzen Pfeil die Blokkierrichtung und durch einen weißen Pfeil die Freigaberichtung angedeutet sind. Die Kopplungshülse 75 ist mit dem Fortsatz 49' drehfest verbunden. Ein in den Fortsatz 49' eingeleitetes Torsionsmoment wird also auf die Kopplungshülse 75 übertragen.

Die weiteren Teile der dritten Blockiereinrichtung 77 werden anhand von Figur 3 näher erläutert.

Figur 3 zeigt lediglich einen Teil der Armauflage 13, nämlich die dritte Blockiereinrichtung 77. Die zweite Blockiereinrichtung 31 in hier aus Gründen der besseren Übersichtlichkeit ebenso wie die weiteren Teile der Armstütze 21 weggelassen.

Die dritte Blockiereinrichtung 77 umfaßt also die Kopplungshülse 75, den mit ihr und dem Lager 81 gekoppelten Drehhebel 79, mit dem ein Betätigungsarm 83 gekoppelt ist, an dessen dem Lager 81 gegenüberliegenden Ende ein dritter Blockierbacken 85 befestigt ist. Dieser wirkt auf ein Widerlager, hier auf ein als Zahnprofil 87 ausgebildetes Zahnelement. Das Zahnprofil ist als runde Zahnstange ausgebildet, auf dessen Außenfläche eine Profilierung beziehungsweise Zähne vorgesehen sind, die mit dem dritten Blockierbacken 85 zusammenwirken. Die Mittelachse des Zahnprofils 87 fällt hier mit der Drehachse 19 zusammen, die anhand der Figuren 1 und 2 bereits erläutert wurde. Bei einer Auf- und Abbewegung der Armauf lage 13 in Richtung der Drehachse 19, also in Richtung einer dritten Bewegungsachse der Armauflage 13, die durch einen Doppelpfeil 89 angedeutet ist, kann die dritte Blockiereinrichtung 77 in einen Freigabe- und in eine Blockierposition verlagert werden, was durch einen Doppelpfeil 91 angedeutet ist. Auch hier ist durch eine schwarze Pfeilspitze die Blockierstellung und durch eine weiße Pfeilspitze die Freigabestellung angedeutet. Das heißt, wenn der dritte Blockierbacken 85 mit dem Zahnprofil 87 in Eingriff gebracht wird, ist eine Auf- und Abbewegung der Armstütze 13 in Richtung des Doppelpfeils 89 ausgeschlossen. Der dritte Blockierbacken 85 wird mit der Kraft des Federelements 47 gegen das Zahnprofil 87 gedrückt.

Bei dem hier dargestellten Ausführungsbeispiel des Arztstuhls 1 sind die drei Blockiereinrichtungen 29, 31 und 77 über die Verstellwelle 49 beziehungsweise deren Fortsetzung 49' starr miteinander gekoppelt. Das heißt, alle Blockiereinrichtungen werden gleichzeitig in die Entriegelungs- und in die Verriegelungs- beziehungsweise Blockier-Position verlagert. Die Blockierbacken 33, 63 und 85 der Blockiereinrichtungen 29, 31 und 77 werden durch die Kraft des Federelements 47 bei freigegebenen Handhebel 43 in ihre Blockierposition verlagert. Es ergibt sich damit ein besonders einfacher Aufbau. Es ist jedoch selbstverständlich möglich, das Federelement 49 einer anderen Blockiereinrichtung als der ersten Blockiereinrichtung 29 zuzuordnen oder bei allen Blockiereinrichtungen ein Federelement vorzusehen. In jedem Fall ist ein beliebiges elastisches Element als Federelement einsetzbar. Bevorzugt wird ein als Schraubenfeder ausgebildetes Federelement, das außen und innen gekammert ist, also beispielsweise in einem Doppelrohr bestehend aus zwei konzentrischen Rohren gelagert ist. Bei einer derartigen Ausgestaltung ist auch bei einem Federbruch eine Restkraft gewährleistet, die eine Verriegelung der Blockierbacken mit den zugehörigen Zahnelementen sicherstellt. Bei einem Federbruch kann ein gebrochenes Federende sich auf der nächstfolgenden Federwicklung abstützen und so die Restkraft gewährleisten. Besonders sicher ist ein Formschluß zwischen den Blockierbacken 33, 63 und 85 mit den Zahnelementen beziehungsweise mit der Zahnstange 59, dem Zahnkranz 61 und/oder dem Zahnprofil 87, wenn die Blockierbacken ihrerseits mit einer Zahnung versehen sind, die in die Zahnung der Zahnelemente eingreift. In einem solchen Fall ist besonders hoher Sicherheit gewährleistet, daß eine einmal eingestellte Blockierung der Armauflage 63 in einer gewünschten Position während der Behandlung eines Patienten eingehalten wird, selbst wenn es zu einem Bruch des Federelements 47 kommen sollte.

Wird also die Verstell- und Fixiereinrichtung 27 mit den drei anhand der Figuren 2 und 3 erläuterten Blockiereinrichtungen 29, 31 und 77 versehen, so kann mit Hilfe des Handhebels 43 eine Entriegelung aller Blockiereinrichtungen gleichzeitig vorgesehen werden. Der Arzt kann die Armauflage 13 dann in die gewünschte Position verlagern. Dabei ist der Handhebel 43 so einfach zu bedienen, daß beispielsweise ein Zahnarzt ein Instrument, wie zum Beispiel einen Bohrer nicht aus der Hand zu legen braucht, während er eine Verstellung der Armauflage 13 durchführt. Wenn die gewünschte Arbeitsposition erreicht ist, wird der Handhebel 43 losgelassen und durch die Kraft des Federelements 47 in seine Verriegelungsposition verlagert. Dadurch verfahren alle drei Blockiereinrichtungen gleichzeitig in ihre Verriegelungsposition, so daß eine Bewegung der Armauflage 13 in allen drei möglichen Achsrichtungen (Längsbewegung, Schwenkbewegung, Höhenverlagerung) blockiert ist.

Das anhand der Figuren 1 bis 3 erläuterte Ausführungsbeispiel des Arztstuhls ist rein mechanisch in drei Achsen verstellbar und mit Hilfe der Einrichtung 27 in der gewünschten Position fixierbar. Es ist auch möglich, bei Betätigung des Handhebels 43 eine elektrische Verstellung zu aktivieren und durch Loslassen des Handhebels 43 zu deaktivieren und gleichzeitig die Armauflage 13 in der erreichten Position zu blockieren beziehungsweise zu fixieren. Auch in diesem Fall ist es also auf einfache Weise möglich, eine optimale Arbeitsposition der Armauflage 13 einzustellen und in Arbeitspausen die Armauflage ohne weiteres in eine Ruheposition zurückzufahren, wie sie beispielsweise durch die gestrichelte Position in Figur 1 angedeutet ist.

Es ist ohne weiteres möglich, in die Tragsäule 15 eine bekannte Gasdruckfeder als Gewichtsausgleich zu integrieren, um die Höhenverstellung in Richtung des Doppelpfeils 17 der Armauflage 13 zu erleichtern. Zusätzlich kann vorgesehen werden, die Armauflage 13 durch eine weitere Gasdruckfeder in die Ruheposition zu drängen, sobald der Handhebel 43 in die Entriegelungsposition verbracht wird. Die hier beschriebene Verstell- und Fixiereinrichtung 27 kann also auf einfache Weise mit herkömmlichen Hilfsmitteln zur Verstellung (Elektromotoren) oder zum Gewichtsausgleich (Gasdruckfeder oder beispielsweise Gegengewichte) kombiniert werden. In allen Fällen ist gewährleistet, daß auf einfache Weise eine Verstellung und Fixierung der Armauflage 13 bei hoher Sicherheit für den Patienten und den Arzt möglich ist.

Die Erläuterungen zu den Figuren 2 und 3 lassen erkennen, daß mit Hilfe der vorzugsweise als Vielkeilwelle ausgebildeten Verstellwelle 49 beziehungsweise deren Fortsetzung 49' mindestens zwei Blockiereinrichtungen miteinander gekoppelt werden können. Es kann also eine Bewegung einer Armauflage 13 eines Arztstuhls 2 in mindestens zwei Achsen blockiert beziehungsweise freigegeben werden. Denkbar ist es beispielsweise, eine Längsverschiebung des Stützelements 21a und eine Höhenverstellung der Armauflage 13 mit den Blockiereinrichtungen 29 und 77 synchron, das heißt gleichzeitig freizugeben beziehungsweise zu blockieren. Möglich ist es auch, die der Schwenkbewegung zugeordnete zweite Blokkiereinrichtung 31 und die der Höhenverstellung zugeordnete dritte Blockiereinrichtung 77 über die Fortsetzung 49' der Verstellwelle 49 zu koppeln und hier gleichzeitig eine Freigabe- beziehungsweise Blockierung zu gewährleisten. Der Arztstuhl 1 kann also bezüglich der Verstell- und Fixiereinrichtung 27 variiert werden, wobei jeweils eine Verstellbarkeit der Armauflage 13 bezüglich zweier Achsen gleichzeitig freigeben beziehungsweise blockiert werden kann. Besonders bewährt hat sich jedoch die Möglichkeit, die Verstellbarkeit der Armauflage bezüglich aller drei Achsen, also bezüglich der Längsverschiebung des Stützelements 21a bezüglich der Drehbewegung um die Drehachse 19 und bezüglich der Höhenverstellung gleichzeitig zu beeinflussen, das heißt synchron freizugeben beziehungsweise zu blockieren.

## Patentansprüche

1. Arztstuhl (1) mit mindestens einer an diesem angebrachten, in mindestens zwei Achsen verstellbaren Armauflage (13) sowie mit einer Verstell- uns Fixiereinrichtung (27) mit einer ersten und zweiten Blockiereinrichtung (29, (31), wobei die einer Bewegung bezüglich einer ersten Achse, vorzugsweise einer Längsverschiebung der Armauflage (13) zugeordnete erste Blockiereinrichtung (29) mit einer federbelasteten, eine definierte Blockierkraft aufbauenden ersten Blockierbacke (33) und die einer Bewegung bezüglich einer zweiten Achse, vorzugsweise einer Drehbewegung der Armauflage (13) zugeordnete zweite Blockiereinrichtung (31) mit einer federbelasteten, eine definierte Blockierkraft aufbauenden zweiten Blockierbacke (63) Versehen ist, **dadurch gekennzeichnet, dass** die Blockiereinrichtungen (29,31) so gekoppelt sind, dass die Bewegung bezüglich der beiden Achsen gleichzeitig blockiert und freigegeben wird, und dass die Blockiereinrichtungen (29,31) über einen gemeinsamen Handhebel (43) aktiviert und deaktiviert werden.

2. Arztstuhl nach Anspruch 1, **dadurch gekennzeichnet, daß** die Verstell- und Fixiereinrichtung (27) eine einer Bewegung bezüglich einer dritten Achse, vorzugsweise einer Höhenverstellung der Armauflage (13) zugeordnete dritte Blockiereinrichtung (77) mit einem federbelasteten, eine definierte Blockierkraft aufbauenden dritten Blockierbacke (85) aufweist, die mit der ersten und/oder zweiten Blockiereinrichtung (29,31) so gekoppelt ist, daß die Bewegung der Armauflage (13) bezüglich der dritten Achse gleichzeitig mit der Bewegung bezüglich der ersten oder zweiten Achse blockiert und freigegeben wird.

3. Arztstuhl nach Anspruch 1 oder 2, **dadurch gekennzeichnet, daß** mindestens eine, vorzugsweise alle Blockierbacken (33,63,85) mit einem Zahnelement (Zahnstange (59), Zahnkranz (61), Zahnprofil (87)) zusammenwirken.

4. Arztstuhl nach Ansprüch 3, **dadurch gekennzeichnet, daß** das der ersten Blockiereinrichtung (29) zugeordnete Zahnelement als Zahnstange (59) ausgebildet ist.

5. Arztstuhl nach Ansprüche 3 oder 4, **dadurch gekennzeichnet, daß** das der zweiten Blockiereinrichtung (31) zugeordnete Zahnelement als Zahnkranz (61) ausgebildet ist.

6. Arztstuhl nach einem der vorhergehenden Ansprüche 3 bis 5, **dadurch gekennzeichnet, daß** das der dritten Blockiereinrichtung (77) zugeordnete Zahnelement als Zahnprofil (87) ausgebildet ist.

7. Arztstuhl nach einem der vorhergehenden Ansprüche, **dadurch gekennzeichnet, daß** der Betätigungshebel (43) mit einer Verstellwelle (49) und gegebenenfalls Fortsetzung (49') gekoppelt ist, die mit der ersten und zweiten Blockiereinrichtung (29,31) gekoppelt ist.

8. Arztstuhl nach Anspruch 7, **dadurch gekennzeichnet, daß** die Verstellwelle (49) und gegebenenfalls Fortsetzung (49') als Vielkeilwelle ausgebildet ist.

9. Arztstuhl nach einem der Ansprüche 7 oder 8, **dadurch gekennzeichnet, daß** die Verstellwelle (49) und gegebenenfalls Fortsetzung (49') mindestens ein Kreuzgelenk (69) aufweist.

## Claims

1. A doctor's chair (1) having at least one arm support (13) which is adjustable in at least two axes, and having an adjusting and locating means (27) with a first and a second locking means (29, 31), wherein the first locking means (29) associated with a movement relative to a first axis, preferably a longitudinal displacement of the arm support (13), is provided with a spring-loaded first locking jaw (33) generating a definite locking force and the second locking means (31) associated with a movement relative to a second axis, preferably a rotating movement of the arm support (13), is provided with a spring-loaded second locking jaw (63) generating a definite locking force, **characterized in that** the locking means (29, 31) are so coupled that the movement relative to the two axes is simultaneously blocked and released, and **in that** the locking means (29, 31) are engaged and disengaged via a common hand lever (43).

2. A doctor's chair according to claim 1, **characterized in that** the adjusting and locating means (27) has a third locking means (77) associated with a movement relative to a third axis, preferably a vertical adjustment of the arm support (13), with a spring-loaded locking jaw (85) generating a definite locking force, which third locking means is so coupled to the first and/or second locking means (29, 31) that the movement of the arm support (13) relative to the third axis is blocked and released simultaneously with the movement of the first or second axis.

3. A doctor's chair according to claim 1 or 2, **characterized in that** at least one, preferably all, the locking jaws (33, 63, 85) co-operated with a toothed element (rack (59), ring gear (61), gear-tooth profile (87)).

4. A doctor's chair according to claim 3, **characterized in that** the toothed element associated with the first locking means (29) is formed as a rack (59).

5. A doctor's chair according to claims 3 or 4, **characterized in that** the toothed element associated with the second locking means (31) is formed as a ring gear (61).

6. A doctor's chair according to any one of the preceding claims 3 to 5, **characterized in that** the toothed element associated with the third locking means (77) is formed as a gear-tooth profile (87).

7. A doctor's chair according to any one of the preceding claims, **characterized in that** the actuating lever (43) is coupled to an adjusting shaft (49) and optionally an extension (49') which is coupled to the first and second locking means (29, 31).

8. A doctor's chair according to claim 7, **characterized in that** the adjusting shaft (49) and optionally the extension (49') is formed as of a multiple-spline shaft.

9. A doctor's chair according to either claim 7 or claim 8, **characterized in that** the adjusting shaft (49) and optionally the extension (49') has at least one universal joint (69).

## Revendications

1. Chaise de médecin (1) comprenant au moins un accoudoir (13) fixé à cette dernière et pouvant être réglé sur au moins deux axes, ainsi qu'un dispositif de réglage et de fixation (27) doté d'un premier et d'un second dispositif de blocage (29, 31), sachant que le premier dispositif de blocage (29) associé à un mouvement par rapport à un premier axe, de préférence à un déplacement longitudinal de l'accoudoir (13), est équipé d'une première mâchoire de blocage (33) soumise à l'action d'un ressort et exerçant une force de blocage définie et que le second dispositif de blocage (31) associé à un mouvement par rapport à un second axe, de préférence à une rotation de l'accoudoir (13), est muni d'une seconde mâchoire de blocage (63) soumise à l'action d'un ressort et exerçant une force de blocage définie, **caractérisée en ce que** les dispositifs de blocage (29, 31) sont couplés de telle manière que le mouvement par rapport aux deux axes est bloqué et débloqué simultanément, et **en ce que** les dispositif de blocage (29, 31) sont activés et désactivés par l'intermédiaire d'un levier à main commun (43).

2. Chaise de médecin selon la revendication 1, **caractérisée en ce que** le dispositif de réglage et de fixation (27) comporte un troisième dispositif de blocage (77) associé à un mouvement par rapport à un troisième axe, de préférence à un déplacement en hauteur de l'accoudoir (13), qui est muni d'une troisième mâchoire de blocage (85) soumise à l'action d'un ressort et exerçant une force de blocage définie et qui est accouplé au premier et/ou au second dispositif de blocage (29, 31) de telle façon que le mouvement de l'accoudoir (13) par rapport au troisième axe est bloqué ou débloqué en même temps que le mouvement par rapport au premier ou au second axe.

3. Chaise de médecin selon la revendication 1 ou 2, **caractérisée en ce qu'**au moins une, de préférence toutes les mâchoires de blocage (33, 63, 85) coopèrent avec un élément denté (crémaillère (59), couronne dentée (61), profilé denté (87)).

4. Chaise de médecin selon la revendication 3, **caractérisée en ce que** l'élément denté associé au premier dispositif de blocage (29) est réalisé en tant que crémaillère (59).

5. Chaise de médecin selon la revendication 3 ou 4, **caractérisée en ce que** l'élément denté associé au second dispositif de blocage (31) est réalisé en tant que couronne dentée (61).

6. Chaise de médecin selon l'une des revendications 3 à 5, **caractérisée en ce que** l'élément denté associé au troisième dispositif de blocage (77) est réalisé en tant que profilé denté (87).

7. Chaise de médecin selon l'une des revendications précédentes, **caractérisée en ce que** le levier d'actionnement (43) est accouplé à un arbre de réglage (49) et le cas échéant à un prolongement (49') qui est accouplé au premier et au second dispositif de blocage (29, 31).

8. Chaise de médecin selon la revendication 7, **caractérisée en ce que** l'arbre de réglage (49) et le cas échéant le prolongement (49') est réalisé en tant qu'arbre cannelé à cales multiples.

9. Chaise de médecin selon l'une des revendications 7 ou 8, **caractérisée en ce que** l'arbre de réglage (49) et le cas échéant le prolongement (49') présente au moins un joint universel (69).
